# EUROPÄISCHE PATENTSCHRIFT

(11) **EP 2 246 010 B1**
(45) Veröffentlichungstag und Bekanntmachung des Hinweises auf die Patenterteilung: **06.01.2016**
(21) Anmeldenummer: 10159710.2
(22) Anmeldetag: 13.04.2010
(51) Int. Cl.: A61C 19/00, A61B 19/00, A61L 2/24

(54) **Reinigungs- oder Pflegevorrichtung für medizinische oder dentale Instrumente und Verfahren zum Betrieb einer Reinigungs- oder Pflegevorrichtung**
Cleaning or maintenance device for medical or dental instruments and method for the operation of a cleaning or maintenance device
Appareil de nettoyage ou d'entretien d'instruments médicaux ou dentaires et procédé de fonctionnement d'un appareil de nettoyage ou d'entretien

(30) Priorität: 27.04.2009 EP 09005816
(43) Veröffentlichungstag der Anmeldung: 03.11.2010
(73) Patentinhaber: W & H Dentalwerk Bürmoos GmbH, 5111 Bürmoos (AT)
(72) Erfinder: Baier, Christof, 5111 Bürmoos (AT); Mangelberger, Michael, 5113 St. Georgen (AT); Pruckner, Christian, 1190 Wien (AT)
(74) Vertreter: Benda, Ralf

(56) Entgegenhaltungen:
- DE-A1-102006 008 723
- US-A1- 2002 161 460
- US-A1- 2004 150 525

## Beschreibung

Die vorliegende Erfindung betrifft eine Reinigungs- oder Pflegevorrichtung für medizinische oder dentale Instrumente nach dem Oberbegriff des Anspruchs 1 und ein Verfahren zum Betrieb einer derartigen Reinigungs- oder Pflegevorrichtung nach dem Oberbegriff des Anspruchs 10.

Aus der Patentanmeldung US 2004/0209223 A1 ist eine Reinigungs- oder Pflegevorrichtung für medizinische oder dentale Instrumente bekannt, in deren Reinigungskammer, insbesondere an den Kupplungsfortsätzen zum Anschluss der Instrumente, eine Lese- und Schreibvorrichtung vorgesehen ist. Über die Lese- und Schreibvorrichtung können Daten von Datenspeichern der Instrumente gelesen werden und gegebenenfalls auch Daten auf die Datenspeichern geschrieben werden.

Da eine Reinigungs- oder Pflegevorrichtung verschiedene sich negativ auf den Datenaustausch auswirkende Elemente aufweist, zum Beispiel in der Reinigungs- oder Pflegevorrichtung enthaltene metallische Bauteile, muss der Datenspeicher der Lese- und Schreibvorrichtung räumlich sehr nahe kommen, damit ein gesicherter Datenaustausch zwischen der Lese- und Schreibvorrichtung und dem Datenspeicher stattfinden kann. So müssen der Datenspeicher und die Lese- und Schreibvorrichtung zum Beispiel bei einem Datenaustausch mittels Radiowellen bei einer Frequenz von 125kHz einander auf wenige Zentimeter angenähert werden. Um eine derart knappe Annäherung zu erreichen ist es somit zwingend erforderlich, die zu reinigenden oder zu pflegenden Instrumente in einer genau definierten Position in die Reinigungs- oder Pflegevorrichtung einzubringen. In der Patentanmeldung US 2004/0209223 wird dies insbesondere dadurch erreicht, dass die Instrumente zur Reinigung oder Pflege an die Kupplungsfortsätze anzuschließen sind.

Bei bestimmten Instrumenten, die zum Beispiel kein Kupplungselement aufweisen, oder bei bestimmten Reinigungsvorrichtungen und den von ihnen durchgeführten Verfahren, zum Beispiel bei Sterilisationsverfahren, bei denen die zu sterilisierenden Instrumente oder Gegenstände in Beutel verpackt werden, ist es jedoch nicht möglich die Instrumente mit den Kupplungsfortsätzen zu verbinden. Derartige Instrumente werden oftmals lose oder als Schüttgut in die Reinigungs- oder Pflegevorrichtung eingelegt, womit die Gefahr besteht, dass ein Datenspeicher eines dieser Instrumente zu weit von der Lese- und Schreibvorrichtung entfernt ist und somit kein Datenaustausch erfolgt.

DE 10 2006 008723 A1 offenbart eine Sterilisationsvorrichtung zur Sterilisation medizintechnischer Objekte, die eine RFID-Lese-/Schreibeinrichtung aufweist.

Der vorliegenden Anmeldung liegt daher die Aufgabe zugrunde eine Reinigungs- oder Pflegevorrichtung für medizinische oder dentale Instrumente und ein Verfahren zum Betrieb einer derartigen Reinigungs- oder Pflegevorrichtung zu schaffen, die diesen Nachteil nicht aufweisen und die insbesondere einen zuverlässigen, von der Position, in der das Instrument in den Reinigungs- oder Pflegeraum eingelegt wurde, unabhängigen Energie- und / oder Datenaustausch zwischen dem Datenspeicher und der Lese- und Schreibvorrichtung ermöglichen.

Zur Lösung dieser Aufgabe wird gemäß einem ersten Ausführungsbeispiel eine Reinigungs- oder Pflegevorrichtung für zumindest ein medizinisches oder dentales Instrument vorgeschlagen, die umfasst: Ein Gehäuse, einen im Inneren des Gehäuses angeordneten Reinigungs- oder Pflegeraum zur Aufnahme des zumindest einen medizinischen oder dentalen Instruments, eine Versorgungsvorrichtung zum Einbringen zumindest eines Reinigungs- oder Pflegemediums in den Reinigungs- oder Pflegeraum, eine am Gehäuse vorgesehene, mit dem Reinigungs- oder Pflegeraum verbundene und durch einen Deckel verschließbare Öffnung zum Einbringen des zumindest einen medizinischen oder dentalen Instrument in den Reinigungs- oder Pflegeraum und zum Entnehmen des zumindest einen medizinischen oder dentalen Instruments aus dem Reinigungs- oder Pflegeraum und eine am Deckel oder an der Öffnung vorgesehen Spule oder Antenne einer Lese- und / oder Schreibvorrichtung, welche dazu ausgebildet ist, einen durch die Öffnung bewegbaren Datenspeicher mittels elektromagnetischer Signale im Radiowellenbereich berührungslos auszulesen und / oder zu beschreiben.

Gemäß einem zweiten, alternativen Ausführungsbeispiel wird eine Reinigungs- oder Pflegevorrichtung für zumindest ein medizinisches oder dentales Instrument vorgeschlagen, die umfasst: Ein Gehäuse mit einer Frontseite, einen im Inneren des Gehäuses angeordneten Reinigungs- oder Pflegeraum zur Aufnahme des zumindest einen medizinischen oder dentalen Instruments, eine Versorgungsvorrichtung zum Einbringen zumindest eines Reinigungs- oder Pflegemediums in den Reinigungs- oder Pflegeraum, eine an der Frontseite des Gehäuses vorgesehene und mit dem Reinigungs- oder Pflegeraum verbundene Öffnung zum Einbringen des zumindest einen medizinischen oder dentalen Instrument in den Reinigungs- oder Pflegeraum und zum Entnehmen des zumindest einen medizinischen oder dentalen Instruments aus dem Reinigungs- oder Pflegeraum und einen die Frontseite überragenden Vorsprung mit einer Spule oder Antenne einer Lese- und / oder Schreibvorrichtung, welche dazu ausgebildet ist, einen durch die Öffnung bewegbaren Datenspeicher mittels elektromagnetischer Signale im Radiowellenbereich berührungslos auszulesen und / oder zu beschreiben. Der Vorsprung ist zum Beispiel als fest mit der Reinigungs- oder Pflegevorrichtung verbundenes Element ausgebildet, insbesondere als Teil des Gehäuses.

Durch das Vorsehen der Spule oder Antenne der Lese- und / oder Schreibvorrichtung an der Öffnung oder am Deckel oder an dem die Frontseite überragenden Vorsprung wird erreicht, dass während des Einbringens des Datenspeichers in den Reinigungs- oder Pflegeraum oder während des Entnehmens des Datenspeichers aus dem Reinigungs- oder Pflegeraum und / oder während des Vorbeiführens des Datenspeichers am Deckel oder am Vorsprung jeder Datenspeicher mit ausreichender Nähe an der Spule oder Antenne der Lese- und / oder Schreibvorrichtung vorbeigeführt wird, so dass zuverlässig ein Datenaustausch und gegebenenfalls auch eine Energieübertragung zwischen dem Datenspeicher und der Lese- und / oder Schreibvorrichtung erfolgt, unabhängig von der Position, in der das Instrument in den Reinigungs- oder Pflegeraum eingelegt wird oder wurde. Ein weiterer Vorteil der beiden Ausführungsbeispiele besteht darin, dass die Spule oder Antenne außerhalb des Reinigungs- oder Pflegeraums und somit weiter entfernt von sich negativ auf den Datenaustausch auswirkenden Elementen angeordnet ist.

Der Datenspeicher ist Teil eines RFID-Chips oder eines RFID-Etikett, das zusätzlich eine Spule oder Antenne umfasst. Der Datenspeicher ist entweder als Lesespeicher (ROM) oder als Lese- und Schreibspeicher (RAM) ausgebildet. Der Datenspeicher ist wahlweise an einem medizinischen oder dentalen Instrument, an einem Träger für ein medizinisches oder dentales Instrument oder an einem Behältnis für ein medizinisches oder dentales Instrument befestigt.

Die Lese- und / oder Schreibvorrichtung weist ebenfalls eine Spule oder Antenne auf, so dass eine berührungslose, insbesondere induktive, Datenübertragung zwischen dem Datenspeicher und der Lese- und / oder Schreibvorrichtung möglich ist. Bevorzugt ist der Datenspeicher oder der RFID-Chip oder das RFID-Etikett als passives Element ausgebildet, d.h. es hat keine eigene Energiequelle oder keinen eigenen Energiespeicher. In diesem Fall erhält der Datenspeicher die für den Betrieb benötigte elektrische Energie auch induktiv von der Lese- und / oder Schreibvorrichtung über die vorgenannten Spulen oder Antennen. Zu diesem Zweck und zur eigenen Energieversorgung ist die Lese- und / oder Schreibvorrichtung mit einer Energiequelle elektrisch verbunden. Alternativ ist es selbstverständlich auch möglich, den RFID-Chip oder das RFID-Etikett als aktives Element mit einem eigenen Energiespeicher auszubilden, zum Beispiel einer Batterie oder einer Kapazität.

Als Reinigungs- oder Pflegevorrichtung im Sinne des vorliegenden Schriftsatzes wird jede Vorrichtung bezeichnet, die zumindest ein Reinigungs- oder Pflegemittel in den Reinigungs- oder Pflegeraum oder an ein medizinisches oder dentales Instrument abgibt, um dieses zu reinigen, zu desinfizieren, zu sterilisieren, von Verschmutzungen oder Mikroorganismen zu befreien oder zu pflegen, insbesondere Sterilisatoren, Autoklaven, Thermodesinfektoren. Als Reinigungs- oder Pflegemittel können zum Beispiel eine Flüssigkeit, insbesondere Heiß- oder Kaltwasser, Dampf, Desinfektions- oder Sterilisationsmittel, ein Gas, zum Beispiel Druckluft, oder ein Schmiermittel, zum Beispiel natürliche oder synthetische Schmieröle, verwendet werden. Die Reinigungs- oder Pflegemittel werden je nach Art des Instruments an dessen Außenseite oder in dessen Inneres, auf das gesamte Instrument oder nur auf bestimmte Bauteile des Instruments abgegeben.

Als zu reinigende medizinische oder dentale Instrumente im Sinne des vorliegenden Schriftsatzes werden insbesondere Hand- und Winkelstücke zum Antrieb verschiedenster Werkzeuge, wie rotierender Bohrer, Zahnsteinentfernungswerkzeuge, Feilen, Sägen, Reamer usw., Handstücke zur Abgabe von medizinischen Materialien, wie zum Beispiel Füllmittel oder Anästhetika, Funktionshandstücke zur Abgabe von Licht, Wasser oder Luft und nicht angetriebene Handinstrumente, wie zum Beispiel medizinische Spiegel, Sonden, Trokare, die im Vorherstehenden genannten oder andere Werkzeuge, Endoskope und dergleichen verstanden.

Gemäß einem Ausführungsbeispiel umgibt die Spule oder Antenne zumindest einen Abschnitt des Außenrandes der Öffnung. Bevorzugt umgibt die Spule oder Antenne die Öffnung kreisförmig oder elliptisch. Durch diese Anordnung wird sichergestellt, dass der Datenspeicher und die Spule oder Antenne einander beim Durchführen des Datenspeichers durch die Öffnung oder beim Bewegen in Richtung der Öffnung oder beim Wegbewegen von der Öffnung besonders nahe kommen.

Gemäß einem anderen Ausführungsbeispiel ist die Spule oder Antenne im oder am Gehäuse vorgesehen, wodurch sie den Durchmesser der Öffnung nicht verkleinert und somit den Anwender beim Einbringen oder Entnehmen der Instrumente nicht stört. Bevorzugt ist die Spule oder Antenne in einer Aufnahme oder in einem Rücksprung des Gehäuses angeordnet.

Gemäß einem Ausführungsbeispiel ist der Deckel relativ zur Öffnung bewegbar und weist, wenn er die Öffnung verschließt, eine dem Reinigungs- oder Pflegeraum zugewandte, erste Seite auf, wobei die Spule oder Antenne an dieser ersten Seite angeordnet ist. Durch diese Anordnung wird sichergestellt, dass der Datenspeicher und die Spule oder Antenne einander beim Vorbeiführen des Datenspeichers am geöffneten Deckel besonders nahe kommen.

Gemäß einem Ausführungsbeispiel ist die Lese- und / oder Schreibvorrichtung zur induktiven Energieübertragung auf den Datenspeicher ausgebildet. Damit ist es möglich, den Datenspeicher oder den RFID-Chip oder das RFID-Etikett als passives Element ohne eigene Energiequelle oder ohne eigenen Energiespeicher auszubilden, wodurch es zum Beispiel widerstandsfähiger gegen bei der Reinigung oder Pflege herrschende Bedingungen ist, insbesondere gegenüber dem Vorhandensein von Wasserdampf.

Gemäß einem weiteren Ausführungsbeispiel ist die Lese- und / oder Schreibvorrichtung mit einer Steuervorrichtung verbunden, die ausgebildet, auf Basis der von dem Datenspeicher ausgelesenen Daten die Reinigungs- oder Pflegevorrichtung zu betreiben. Damit ist eine automatisierte Reinigung oder Pflege realisierbar, bei der automatisch die für das jeweilige Instrument passenden Parameter oder Reinigungs- oder Pflegeverfahren oder Verfahrensschritte ausgewählt werden. Dazu sind gemäß einer bevorzugten Ausführungsform am Datenspeicher Daten gespeichert, die das Instrument definieren, dem der Datenspeicher zugeordnet ist, zum Beispiel eine Instrumentennummer oder ein Instrumentencode. Die Steuervorrichtung ist demgemäß ausgebildet, die von der Lese- und / oder Schreibvorrichtung ausgelesenen und an die Steuervorrichtung geleiteten Daten des Datenspeichers den entsprechenden, in einem Speicherelement der Steuervorrichtung gespeicherten Parametern oder Reinigungs- oder Pflegeverfahren oder Verfahrensschritten zuzuordnen und die Reinigungs- oder Pflegevorrichtung entsprechend zu betreiben. Alternativ sind am Datenspeicher Daten gespeichert, die die Parameter oder Reinigungs- oder Pflegeverfahren oder Verfahrensschritte des Instruments definieren, dem der Datenspeicher zugeordnet ist, zum Beispiel die maximale Verfahrenstemperatur, der maximale Verfahrensdruck, die Zusammensetzung, Einwirkdauer oder Menge einer Reinigungsflüssigkeit oder die Art eines Pflegemittels. Die Steuervorrichtung ist demgemäß ausgebildet, die Reinigungs- oder Pflegevorrichtung entsprechend den von der Lese- und / oder Schreibvorrichtung ausgelesenen und an die Steuervorrichtung geleiteten Daten des Datenspeichers zu betreiben.

Gemäß einem weiteren Ausführungsbeispiel ist die Lese- und / oder Schreibvorrichtung mit einer Steuervorrichtung verbunden, die ausgebildet ist, Daten über ein von der Reinigungs- oder Pflegevorrichtung durchgeführtes Reinigungs- oder Pflegeverfahren an die Lese- und / oder Schreibvorrichtung zu übertragen, so dass die Lese- und / oder Schreibvorrichtung diese Daten auf den Datenspeicher überträgt. Die Daten über ein durchgeführtes Reinigungs- oder Pflegeverfahren umfassen zum Beispiel Angaben über Verfahrensparameter, insbesondere Temperatur- oder Druckangaben oder Zeitdauern, oder über durchgeführte oder nicht durchgeführte Verfahrensschritte. Zur Ermittlung dieser Daten ist die Steuervorrichtung mit entsprechenden Sensoren oder Detektoren versehen, zum Beispiel Temperatur- oder Drucksensoren, welche die Daten an die Steuervorrichtung leiten. Die Steuervorrichtung überträgt diese Daten an die Lese- und / oder Schreibvorrichtung.

Zu einem späteren Zeitpunkt, zum Beispiel vor der Verwendung des Instruments, können diese Daten von einer beliebigen, mit dem Datenspeicher verbindbaren Steuervorrichtung ausgelesen und verwertet werden. Durch Vergleich der Daten über ein durchgeführtes Reinigungs- oder Pflegeverfahren mit vorgegebenen Sollwerten kann insbesondere sicher gestellt werden, dass ein unzureichend gereinigtes Instrument nicht verwendet wird. In vorteilhafter Weise ist somit der dem Instrument zugeordnete Datenspeicher der Träger der Daten und Informationen bezüglich eines durchgeführten Reinigungs- oder Pflegeverfahrens.

Gemäß einem bevorzugten Ausführungsbeispiel ist die Steuervorrichtung ausgebildet, das Reinigungs- oder Pflegeverfahren zu überwachen und nur dann Daten über das Reinigungs- oder Pflegeverfahren an die Lese- und / oder Schreibvorrichtung zu übertragen, wenn die Reinigungs- oder Pflegevorrichtung das Reinigungs- oder Pflegeverfahren tatsächlich ausführt. Die Überwachung des Reinigungs- oder Pflegeverfahrens erfolgt zum Beispiel über Sensoren oder Detektoren der Steuervorrichtung, die Verfahrensparameter des Reinigungs- oder Pflegeverfahrens überwachen, zum Beispiel Temperatur-, Durchfluss- oder Druckwerte oder Zeitdauern. Nur wenn zumindest ein Verfahrensparameter einen vorgegebenen Wert erreicht oder überschreitet, überträgt die Steuervorrichtung Daten über das Reinigungs- oder Pflegeverfahren an die Lese- und / oder Schreibvorrichtung.

Gemäß einem Ausführungsbeispiel umfasst eine Reinigungs- oder Pflegeeinheit eine in dem Schriftstück beschriebene Reinigungs- oder Pflegevorrichtung und zumindest ein durch die Öffnung der Reinigungs- oder Pflegevorrichtung bewegbares und mit einem durch die Lese- und / oder Schreibvorrichtung auslesbaren und / oder beschreibbaren Datenspeicher versehenes Element, wobei das Element als medizinisches oder dentales Instrument, als Träger für ein medizinisches oder dentales Instrument oder als Behältnis für ein medizinisches oder dentales Instrument ausgebildet ist. Der Datenspeicher muss somit nicht zwingend am zu reinigenden oder zu pflegenden Instrument befestigt sein, er kann auch auf einem Träger oder Behältnis für ein derartiges Instrument vorgesehen sein. Der Träger oder das Behältnis sind zum Beispiel als Kassette, insbesondere als Sterilisationskassette, als Tray oder als Beutel, insbesondere als Kunststoffbeutel für die Sterilisation, ausgebildet. Eine derartige Ausführung ist insbesondere dann von Vorteil, wenn mehrere Instrumente als ein zusammengehöriges Instrumentenset gemeinsam in einem Träger oder Behältnis aufbewahrt, gemeinsam verwendet und gemeinsam gereinigt oder gepflegt werden. Damit ist es nicht nötig, jedes Instrument mit einem eigenen Datenspeicher zu versehen.

Ein Verfahren zum Betrieb einer in dem Schriftstück beschriebenen Reinigungs- oder Pflegevorrichtung oder einer in dem Schriftstück beschriebenen Reinigungs- oder Pflegeeinheit umfasst den Schritt, dass während des Bewegens des Datenspeichers durch die am Gehäuse vorgesehene Öffnung oder in Richtung der Öffnung oder von der Öffnung weg oder während des Vorbeiführens des Datenspeichers am geöffneten Deckel oder während des Vorbeiführens des Datenspeichers an dem die Frontseite überragenden Vorsprung die Lese- und / oder Schreibvorrichtung den Datenspeicher berührungslos ausliest und / oder beschreibt. Bevorzugt erfolgt das Bewegen des Datenspeichers durch die am Gehäuse vorgesehene Öffnung oder in Richtung der Öffnung oder von der Öffnung weg oder das Vorbeiführen des Datenspeichers am geöffneten Deckel oder das Vorbeiführen des Datenspeichers an dem die Frontseite überragenden Vorsprung manuell.

Gemäß einem Ausführungsbeispiel wird der Datenspeicher beim Einbringen des Datenspeichers in den Reinigungs- oder Pflegeraum ausgelesen und beim Entnehmen des Datenspeichers aus dem Reinigungs- oder Pflegeraum beschrieben.

Gemäß einem weiteren Ausführungsbeispiel überwacht die Steuervorrichtung ein von der Reinigungs- oder Pflegevorrichtung ausgeführtes Reinigungs- oder Pflegeverfahren und überträgt nur dann Daten über das Reinigungs- oder Pflegeverfahren an die Lese- und / oder Schreibvorrichtung, wenn die Reinigungs- oder Pflegevorrichtung das Reinigungs- oder Pflegeverfahren tatsächlich ausführt.

Die Erfindung wird nachfolgend anhand eines bevorzugten Ausführungsbeispiels und Bezug nehmend auf die beigefügten Zeichnungen erläutert:
Figur 1 zeigt eine perspektivische Ansicht eines ersten Ausführungsbeispiels einer Reinigungs- oder Pflegevorrichtung mit einer Gehäuseöffnung und einer an der Öffnung angeordneten Spule.
Figur 2 zeigt in einer schematischen Darstellung einen Schnitt durch die Reinigungs- oder Pflegevorrichtung der Figur 1.
Figur 3 zeigt eine perspektivische Ansicht eines zweiten Ausführungsbeispiels einer Reinigungs- oder Pflegevorrichtung mit einem die Frontseite überragenden Vorsprung und einer darin vorgesehenen Antenne.
Figur 4 zeigt eine perspektivische Ansicht eines dritten Ausführungsbeispiels einer Reinigungs- oder Pflegevorrichtung mit einem eine Gehäuseöffnung verschließenden Deckel und einer an dem Deckel angeordneten Spule.
Figur 5A zeigt eine perspektivische Ansicht eines Behältnisses mit einem Datenspeicher für zu reinigende oder zu pflegende Instrumente in Form eines Kunststoffbeutels.
Figur 5B zeigt eine perspektivische Ansicht eines Trägers mit einem Datenspeicher für zu reinigende oder zu pflegende Instrumente in Form einer Kassette, mit einem ersten Instrument ohne Datenspeicher und einem zweiten Instrument mit einem Datenspeicher.

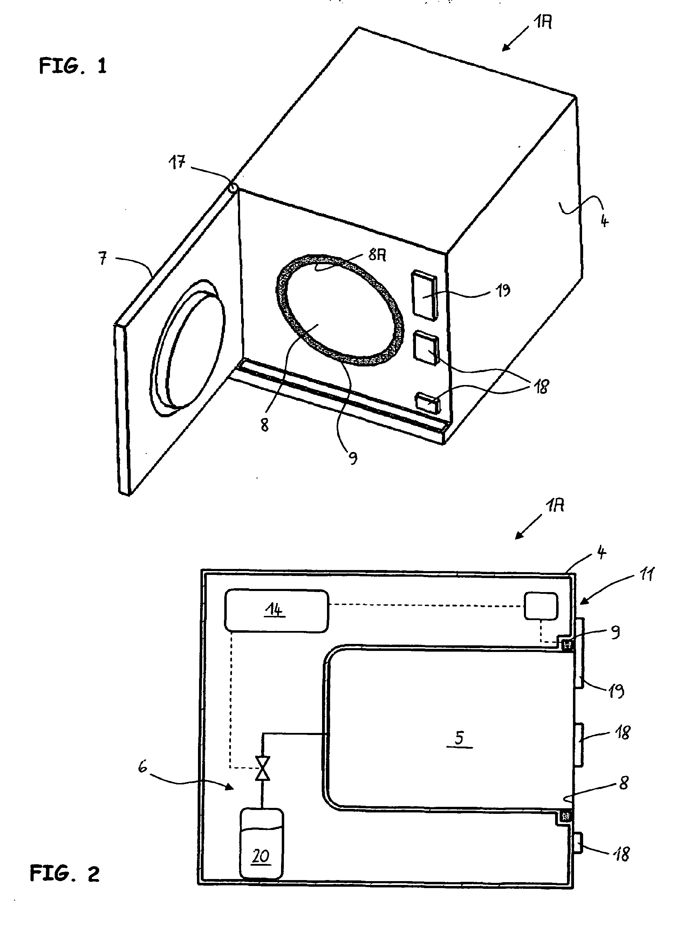

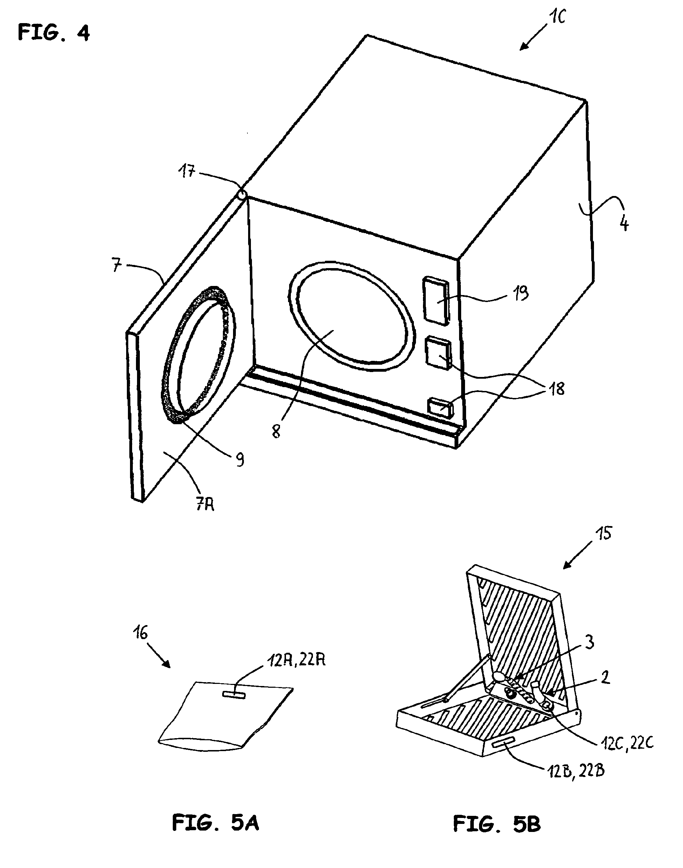

Die Figuren 1 und 2 zeigen ein erstes Ausführungsbeispiel einer Reinigungs- oder Pflegevorrichtung 1A in Form eines Sterilisators, insbesondere eines Dampfsterilisators. Der Sterilisator 1A umfasst ein Außengehäuse 4 mit mehreren Wänden und einen Deckel 7, der relativ zu den Wänden beweglich ist, zum Beispiel durch ein Scharnier oder Drehgelenk 17. An einer der Wände des Gehäuses 4 ist eine Öffnung 8 vorgesehen, die mit einem Reinigungs- oder Pflegeraum 5 des Sterilisators 1A verbunden ist. Durch diese Öffnung 8 kann ein zu reinigendes Instrument 2, 3 oder ein Träger 15 (Figur 5B) für ein medizinisches oder dentales Instrument 2, 3 oder ein Behältnis 16 (Figur 5A) für ein medizinisches oder dentales Instrument 2, 3 in den Reinigungs- oder Pflegeraum 5 eingebracht oder daraus entnommen werden.

Am Gehäuse 4 sind ein oder mehrere Bedienelemente 18 vorgesehen, die zum Beispiel zur Auswahl unterschiedlicher Betriebsprogramme oder zur Einstellung von Betriebsparametern dienen. Eine Anzeige 19 zeigt die ausgewählten Betriebsprogramme oder Betriebsparameter oder sonstige für den Betrieb des Sterilisators 1A relevante Daten oder Kennwerte an.

Im Inneren des Gehäuses 4 sind neben dem Reinigungs- oder Pflegeraum 5 eine Versorgungsvorrichtung 6 zum Einbringen zumindest eines Reinigungs- oder Pflegemediums in den Reinigungs- oder Pflegeraum 5 und eine Steuervorrichtung 14 vorgesehen. Der Reinigungs- oder Pflegeraum 5 des Sterilisators 1A ist als Druckkessel ausgeführt, der zum Beispiel mit der Versorgungsvorrichtung 6 verbundene Anschlüsse aufweist, an die Instrumente 2, 3 anschließbar sind, und / oder in den der Träger 15 oder der Behälter 16 für die Instrumente 2, 3 einbringbar sind.

Die Versorgungsvorrichtung 6 umfasst zum Beispiel einen oder mehrere Behälter 20 für Reinigungs- und / oder Pflegemittel, Ventile, Pumpen, Medienleitungen und -rohre, einen Verdampfer, einen oder mehrere Anschlüsse an externe Medienquellen, einen Anschluss an eine Energiequelle, Behälter für verbrauchte Reinigungs- und / oder Pflegemittel, etc.

Die Steuervorrichtung 14 umfasst zum Beispiel eine oder mehrere Steuerschaltungen, einen Mikrocontroller oder Sensoren zur Detektion von Betriebsparametern. Die Steuervorrichtung 14 ist über Steuerleitungen mit der Versorgungsvorrichtung 6, mit den Bedienelementen 18, mit der Anzeige 19 und mit einer Lese- und / oder Schreibvorrichtung 11 verbunden. Die Steuervorrichtung 14 empfängt von diesen Bauteilen Signale und / oder sendet an diese Bauteile Signale und steuert und / oder regelt damit den Betrieb der Reinigungs- oder Pflegevorrichtung 1A, insbesondere den Ablauf der Reinigungs- und / oder Pflegeverfahren und die Einhaltung der Kenngrößen während der Reinigungs- und / oder Pflegeverfahren.

Die Lese- und / oder Schreibvorrichtung 11 umfasst zumindest eine Spule 9, die zum Beispiel aus einem elektrisch leitfähigem Spulendraht gebildet ist oder als gedruckte oder aufgedampfte Folie ausgebildet ist. Die Lese- und / oder Schreibvorrichtung 11 dient dazu, den Datenspeicher 12A, 12B, 12C eines RFID-Etiketts 22A, 22B, 22C induktiv mittels elektromagnetischer Signale im Radiowellenbereich berührungslos auszulesen und / oder zu beschreiben und gegebenenfalls Energie auf das RFID-Etikett 22A, 22B, 22C zu übertragen. Die Datenspeicher 12A, 12B, 12C sind entweder direkt an Instrumenten 2, 3 befestigt oder sie sind an einem Träger 15 für ein medizinisches oder dentales Instrument 2, 3, zum Beispiel einer Kassette, oder an einem Behältnis 16 für ein medizinisches oder dentales Instrument 2, 3, zum Beispiel einem Sterilisationsbeutel, vorgesehen. Die Lese- und / oder Schreibvorrichtung 11 kann bevorzugt weitere Bauteile, zum Beispiel einen, insbesondere bidirektionalen, Signalverstärker für die induktiv übertragbaren Daten, eine Signalverarbeitungseinheit, eine Energiequelle oder einen Anschluss an eine Energiequelle aufweisen. Alternativ sind eines oder mehrere dieser Elemente Teil der Steuervorrichtung 14.

Die Steuervorrichtung 14 ist über eine Steuerleitung mit der Lese- und / oder Schreibvorrichtung 11 verbunden. Die Steuervorrichtung 14 ist ausgebildet, auf Basis der vom Datenspeicher 12A, 12B, 12C ausgelesenen Daten die Reinigungs- oder Pflegevorrichtung 1A, 1B 1C zu betreiben, insbesondere ein Reinigungs- oder Pflegeverfahren auszuwählen oder Parameter für den Betrieb der Reinigungs- oder Pflegevorrichtung 1A automatisch einzustellen.

Die Steuervorrichtung 14 ist bevorzugt ausgebildet, Daten über ein von der Reinigungs- oder Pflegevorrichtung 1A durchgeführtes Reinigungs- oder Pflegeverfahren an die Lese- und / oder Schreibvorrichtung 11 zu übertragen, so dass die Lese- und / oder Schreibvorrichtung 11 diese Daten auf den Datenspeicher 12A, 12B, 12C überträgt, insbesondere auf einen Datenspeicher 12A, 12B, 12C der direkt an einem Instrument 2, 3 befestigt ist. Der am Instrument 2, 3 befestigte Datenspeicher 12A, 12B, 12C dient somit als zentrale Speichereinheit, auf der die für das Instrument 2, 3 relevanten Daten gespeichert sind und die von Lesevorrichtungen beliebiger anderer Geräte abgefragt werden kann, ohne dass diese Geräte untereinander vernetzt sein müssen. Derartige Geräte umfassen insbesondere Steuer-, Versorgungs- oder Antriebsvorrichtungen, an denen die Instrumente 2, 3 anschließbar sind. Von der Lese- und / oder Schreibvorrichtung 11 auf den Datenspeicher 12A, 12B, 12C übertragene Daten umfassen insbesondere Angaben, ob das Instrument 2, 3 gereinigt oder gepflegt wurde, mit welchem Verfahren es gereinigt oder gepflegt wurde, bei welchen Parametern (Temperatur, Druck, etc.) es gereinigt oder gepflegt wurde oder wie oft es in Summe bereits gereinigt oder gepflegt wurde.

Besonders bevorzugt ist die Steuervorrichtung 14 ausgebildet, das von der Reinigungs- oder Pflegevorrichtung 1A durchgeführte Reinigungs- oder Pflegeverfahren zu überwachen und nur dann Daten über das Reinigungs- oder Pflegeverfahren an die Lese- und / oder Schreibvorrichtung 11 zu übertragen, wenn die Reinigungs- oder Pflegevorrichtung 1A das Reinigungs- oder Pflegeverfahren tatsächlich ausführt oder ausgeführt hat.

Gemäß dem in den Figuren 1 und 2 dargestellten Ausführungsbeispiel ist die Spule 9 im Gehäuse 4 oder in der Gehäusewand angeordnet und umgibt den Außenrandes 8A der Öffnung 8 kreisförmig. Sind die Öffnung 8 des Gehäuses und die Aufnahmeöffnung des Reinigungs- oder Pflegeraums 5 durch eine gemeinsame Öffnung gebildet, so kann alternativ die Spule 9 an der Öffnung des Reinigungs- oder Pflegeraums 5 angeordnet sein. Wird ein Datenspeicher 12A, 12B, 12C eines RFID-Etiketts 22A, 22B, 22C durch die Öffnung 8 bewegt, so erfolgt ein induktiver Daten- und / oder Energieaustausch zwischen der Spule 9 und einer Spule des RFID-Etiketts 22A, 22B, 22C.

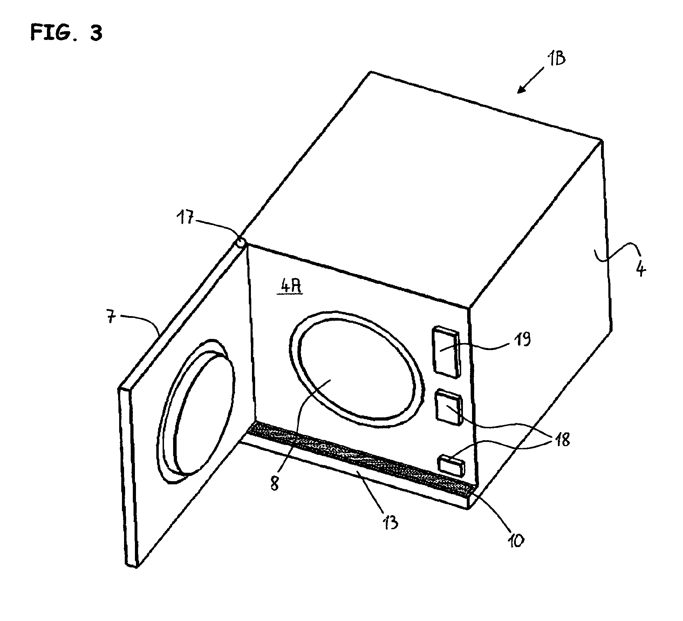

Die in der Figur 3 dargestellte Reinigungs- oder Pflegevorrichtung 1B ist als kombinierte Reinigungs- und Pflegevorrichtung ausgebildet, die bevorzugt die Instrumente 2, 3 mittels zumindest einer desinfizierenden Flüssigkeit reinigt, mittels Wasser spült und gegebenenfalls mittels zumindest eines Schmiermittels pflegt. Der Aufbau der Reinigungs-und Pflegevorrichtung 1B ist im Wesentlichen ähnlich wie im Vorherstehenden für die Reinigungs- oder Pflegevorrichtung 1A in den Figuren 1 und 2 beschrieben, wobei gleiche Bauteile mit den gleichen Bezugszeichen versehen sind.

Das Gehäuse 4 der Reinigungs- und Pflegevorrichtung 1B weist eine Frontseite 4A auf, an der die mit dem Reinigungs- oder Pflegeraum 5 verbundene Öffnung 8 zum Einbringen des zumindest einen medizinischen oder dentalen Instruments 2, 3 in den Reinigungs- oder Pflegeraum 5 und zum Entnehmen des zumindest einen medizinischen oder dentalen Instruments 2, 3 aus dem Reinigungs- oder Pflegeraum 5 vorgesehen ist. Ein an der Frontseite 4A vorgesehener Vorsprung 13 überragt die Frontseite 4A. Am Vorsprung 13, insbesondere an einer der Öffnung 8 zugewandten Seite des Vorsprungs 13, ist eine Antenne 10 der Lese- und / oder Schreibvorrichtung 11 vorgesehen, welche dazu ausgebildet ist, einen durch die Öffnung 8 bewegbaren Datenspeicher 12A, 12B, 12C eines RFID-Etiketts 22A, 22B, 22C mittels elektromagnetischer Signale im Radiowellenbereich berührungslos auszulesen und / oder zu beschreiben. Wird der Datenspeicher 12A, 12B, 12C an der Antenne 10 vorbeigeführt, so dient die Antenne 10 dem induktiven Daten- und / oder Energieaustausch zwischen der Lese- und / oder Schreibvorrichtung 11 und einer Spule des RFID-Etiketts 22A, 22B, 22C. Die Antenne 10 ist zum Beispiel als lineare Antenne in Form eines metallischen Drahtes oder Stabes oder als Faltdipol oder als gedruckte oder aufgedampfte Folie ausgebildet.

Die in der Figur 4 dargestellte Reinigungs- oder Pflegevorrichtung 1C ist als Thermodesinfektor ausgebildet, der bevorzugt die Instrumente 2, 3 mittels zumindest einer Reinigungsflüssigkeit reinigt und mittels Dampf desinfiziert. Der Aufbau des Thermodesinfektors 1C ist im Wesentlichen ähnlich wie im Vorherstehenden für die Reinigungs- oder Pflegevorrichtung 1A in den Figuren 1 und 2 beschrieben, wobei gleiche Bauteile mit den gleichen Bezugszeichen versehen sind.

Der relativ zur Öffnung 8 bewegbare Deckel 7 des Thermodesinfektors 1C weist, wenn er die Öffnung 8 verschließt, eine dem Reinigungs- oder Pflegeraum 5 zugewandte, erste Seite 7A auf. An dieser ersten Seite 7A ist eine Spule 9 angeordnet, so dass, wenn ein durch die Öffnung 8 bewegbarer Datenspeicher 12A, 12B, 12C eines RFID-Etiketts 22A, 22B, 22C an der Spule 9 vorbei bewegt wird, ein induktiver Daten- und / oder Energieaustausch zwischen der Spule 9 und einer Spule des RFID-Etiketts 22A, 22B, 22C erfolgt.

Die Figuren 5A und 5B zeigen medizinische Vorrichtungen mit RFID-Etiketten 22A, 22B, 22C, die jeweils eine Spule aufweisen, um mit der Spule 9 oder der Antenne 10 der Reinigungs- oder Pflegevorrichtungen 1A, 1B, 1C mittels elektromagnetischer Signale im Radiowellenbereich berührungslos Daten und / oder Energie auszutauschen. Die Figur 5A zeigt einen Kunststoffbeutel 16 mit einem RFID-Etikett 22A, insbesondere zur Verwendung in Sterilisatoren, in dem ein oder mehrere medizinische oder dentale Instrumente oder Zubehörteile aufnehmbar sind. Die Figur 5B zeigt eine Reinigungskassette 15 mit einem RFID-Etikett 22B. Der Vorteil des mit einem RFID-Etikett 22A versehenen Beutels 16 und der mit einem RFID-Etikett 22B versehenen Kassette 15 liegt darin, dass es hiermit möglich ist, Daten für ein Reinigungs- oder Pflegeverfahren oder den Nachweis einer Reinigung oder Pflege auch für ein Instrument 3 ohne RFID-Etikett zu übertragen. Im Gegensatz dazu weist das als Handstück ausgebildete Instrument 2 ein eigenes RFID-Etikett 22C auf, um mit der Spule 9 oder der Antenne 10 der Reinigungs- oder Pflegevorrichtungen 1A, 1B, 1C berührungslos, induktiv Daten und / oder Energie auszutauschen. Das Handstück 2 kann dabei in einer Kassette oder ohne Kassette in die Reinigungs- oder Pflegevorrichtungen 1A, 1B, 1C eingebracht werden.

Die Erfindung ist nicht auf die dargestellten und beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

So ist es gemäß einem Ausführungsbeispiel möglich, dass mehrere Spulen 9 oder mehrere Antennen 10 oder zumindest eine Spule 9 und eine Antenne 10 an der Öffnung 8 oder am Deckel 7 oder an dem Vorsprung 13 vorgesehen sind. Bevorzugt weisen die Spulen 9 und / oder Antennen 10 unterschiedliche Richtwirkungen auf und / oder sind zur Übertragung unterschiedlicher Frequenzbereiche ausgebildet.

Gemäß einem anderen Ausführungsbeispiel sind an zumindest zwei der drei beschriebenen Positionen (Öffnung 8, Deckel 7, Vorsprung 13) jeweils zumindest eine Spule 9 und / oder eine Antenne 10 vorgesehen. Bevorzugt weisen die Spulen 9 und / oder Antennen 10 wiederum unterschiedliche Richtwirkungen auf und / oder sind zur Übertragung unterschiedlicher Frequenzbereiche ausgebildet.

Beide Ausführungsbeispiele haben den Vorteil, dass durch die räumliche Anordnung und / oder die Mehrzahl an Spulen 9 oder Antennen 10 eine verbesserte Richtwirkung erzielt wird und / oder der Erfassungs- oder Wirkraum für den Daten- und / oder Energietransfer besser definiert oder abgegrenzt wird. Ein besonders gut abgegrenzter Erfassungs- oder Wirkraum wird erzielt, wenn an jeder drei beschriebenen Positionen (Öffnung 8, Deckel 7, Vorsprung 13) jeweils zumindest eine Spule 9 und / oder eine Antenne 10 angeordnet ist.

Gemäß einem weiteren Ausführungsbeispiel ist die bevorzugte Richtwirkung der Spule 9 und / oder Antenne 10, insbesondere wenn diese am Deckel 7 oder am Vorsprung 13 angeordnet sind, in Richtung der Öffnung 8 oder der Frontseite 4A ausgebildet. In vorteilhafter Weise wird dadurch vor der Öffnung 8 ein definierter Erfassungs- oder Wirkraum für den Daten- und / oder Energietransfer geschaffen.

Gemäß einem anderen Ausführungsbeispiel sind an der Reinigungs- oder Pflegevorrichtung 1A, 1B, 1C, insbesondere an der Spule 9 oder Antenne 10 oder um die Spule 9 oder Antenne 10, ein Reflektor oder eine Abschirmvorrichtung vorgesehen. Der Reflektor oder die Abschirmvorrichtung sind zum Beispiel durch einen oder mehrere Stäbe, durch ein Gitter oder durch Folien gebildet, die jeweils leitfähige Metalle umfassen. Der Reflektor oder die Abschirmvorrichtung tragen wiederum zur Schaffung eines definierten Erfassungs- oder Wirkraums für den Daten- und / oder Energietransfer bei und / oder verhindern oder erschweren einen unerwünschten Daten- und / oder Energietransfer mit Datenspeichern außerhalb des Erfassungs- oder Wirkraums.

## Patentansprüche

1. Reinigungs- oder Pflegevorrichtung (1 A, 1 B, 1C) für zumindest ein medizinisches oder dentales Instrument (2, 3), umfassend ein Gehäuse (4), einen im Inneren des Gehäuses (4) angeordneten Reinigungs- oder Pflegeraum (5) zur Aufnahme des zumindest einen medizinischen oder dentalen Instruments (2, 3), eine Versorgungsvorrichtung (6) zum Einbringen zumindest eines Reinigungs- oder Pflegemediums in den Reinigungs- oder Pflegeraum (5) und eine am Gehäuse (4) vorgesehene, mit dem Reinigungs- oder Pflegeraum (5) verbundene und durch einen Deckel (7) verschließbare Öffnung (8) zum Einbringen des zumindest einen medizinischen oder dentalen Instruments (2, 3) in den Reinigungs- oder Pflegeraum (5) und zum Entnehmen des zumindest einen medizinischen oder dentalen Instruments (2, 3) aus dem Reinigungs- oder Pflegeraum (5), wobei am Deckel (7) oder an der Öffnung (8) eine Spule (9) oder eine Antenne (10) einer Lese- und / oder Schreibvorrichtung (11) vorgesehen ist, welche dazu ausgebildet ist, einen durch die Öffnung (8) bewegbaren Datenspeicher (12A, 12B, 12C) mittels elektromagnetischer Signale im Radiowellenbereich berührungslos auszulesen und / oder zu beschreiben, wobei der Deckel (7) relativ zur Öffnung (8) bewegbar ist und, wenn er die Öffnung (8) verschließt, eine dem Reinigungs- oder Pflegeraum (5) zugewandte, erste Seite (7A) aufweist, an welcher die Spule (9) oder Antenne (10) angeordnet ist, **dadurch gekennzeichnet, dass**
die Spule (9) oder Antenne (10) zumindest einen Abschnitt des Außenrandes (8A) der Öffnung (8) umgibt, wobei die Spule (9) oder Antenne (10) die Öffnung (8) kreisförmig oder elliptisch umgibt.

2. Reinigungs- oder Pflegevorrichtung (1 A, 1 B, 1C) für zumindest ein medizinisches oder dentales Instrument (2, 3), umfassend ein Gehäuse (4) mit einer Frontseite (4A), einen im Inneren des Gehäuses (4) angeordneten Reinigungs- oder Pflegeraum (5) zur Aufnahme des zumindest einen medizinischen oder dentalen Instruments (2, 3), eine Versorgungsvorrichtung (6) zum Einbringen zumindest eines Reinigungs- oder Pflegemediums in den Reinigungs- oder Pflegeraum (5), eine an der Frontseite (4A) des Gehäuses (4) vorgesehene und mit dem Reinigungs- oder Pflegeraum (5) verbundene Öffnung (8) zum Einbringen des zumindest einen medizinischen oder dentalen Instruments (2, 3) in den Reinigungs- oder Pflegeraum (5) und zum Entnehmen des zumindest einen medizinischen oder dentalen Instruments (2, 3) aus dem Reinigungs- oder Pflegeraum (5) und einen Deckel (7) zum Verschließen der Öffnung (8), der relativ zu den Wänden des Gehäuses (4) beweglich ist, **dadurch gekennzeichnet, dass**
an einem die Frontseite (4A) überragenden Vorsprung (13) eine Spule (9) oder eine Antenne (10) einer Lese- und / oder Schreibvorrichtung (11) vorgesehen ist, welche dazu ausgebildet ist, einen durch die Öffnung (8) bewegbaren Datenspeicher (12A, 12B, 12C) mittels elektromagnetischer Signale im Radiowellenbereich berührungslos auszulesen und / oder zu beschreiben.

3. Reinigungs- oder Pflegevorrichtung (1A, 1 B, 1 C) nach Anspruch 1 oder 2, **dadurch gekennzeichnet, dass**
mehrere Spulen (9) oder mehrere Antennen (10) oder zumindest eine Spule (9) und eine Antenne (10) an der Öffnung (8) oder am Deckel (7) oder an dem Vorsprung (13) vorgesehen sind, wobei die Spulen (9) und / oder Antennen (10) unterschiedliche Richtwirkungen aufweisen und / oder zur Übertragung unterschiedlicher Frequenzbereich ausgebildet sind oder dass an zumindest zwei der drei Positionen Öffnung (8), Deckel (7) oder Vorsprung (13) jeweils zumindest eine Spule (9) und / oder eine Antenne (10) vorgesehen ist, wobei die Spulen (9) und / oder Antennen (10) wiederum unterschiedliche Richtwirkungen aufweisen und / oder zur Übertragung unterschiedlicher Frequenzbereiche ausgebildet sind.

4. Reinigungs- oder Pflegevorrichtung (1A, 1 B, 1C) nach einem der vorherstehenden Ansprüche, **dadurch gekennzeichnet, dass**
die Spule (9) oder Antenne (10) im oder am Gehäuse (4) vorgesehen ist.

5. Reinigungs- oder Pflegevorrichtung (1A, 1B, 1C) nach Anspruch 1, **gekennzeichnet durch**
einen relativ zur Öffnung (8) bewegbaren Deckel (7), der, wenn er die Öffnung (8) verschließt, eine dem Reinigungs- oder Pflegeraum (5) zugewandte, erste Seite (7A) aufweist, wobei die Spule (9) oder Antenne (10) an dieser ersten Seite (7A) angeordnet ist.

6. Reinigungs- oder Pflegevorrichtung (1A, 1B, 1C) nach einem der vorherstehenden Ansprüche, **dadurch gekennzeichnet, dass**
die Lese- und / oder Schreibvorrichtung (11) zur induktiven Energieübertragung auf den Datenspeicher (12A, 12B, 12C) ausgebildet ist.

7. Reinigungs- oder Pflegevorrichtung (1A, 1B, 1C) nach einem der vorherstehenden Ansprüche, **dadurch gekennzeichnet, dass**
die Lese- und / oder Schreibvorrichtung (11) mit einer Steuervorrichtung (14) verbunden ist, die ausgebildet ist, auf Basis der vom Datenspeicher (12A, 12B, 12C) ausgelesenen Daten die Reinigungs- oder Pflegevorrichtung (1A, 1 B, 1C) zu betreiben.

8. Reinigungs- oder Pflegevorrichtung (1A, 1B, 1C) nach einem der vorherstehenden Ansprüche, **dadurch gekennzeichnet, dass**
die Lese- und / oder Schreibvorrichtung (11) mit einer Steuervorrichtung (14) verbunden ist, die ausgebildet ist, Daten über ein von der Reinigungs- oder Pflegevorrichtung (1A, 1 B, 1 C) durchgeführtes Reinigungs- oder Pflegeverfahren an die Lese- und / oder Schreibvorrichtung (11) zu übertragen, so dass die Lese- und / oder Schreibvorrichtung (11) diese Daten auf den Datenspeicher (12A, 12B, 12C) überträgt.

9. Reinigungs- oder Pflegevorrichtung (1 A, 1 B, 1C) nach Anspruch 8, **dadurch gekennzeichnet, dass**
die Steuervorrichtung (14) ausgebildet ist, das Reinigungs- oder Pflegeverfahren zu überwachen und nur dann Daten über das Reinigungs- oder Pflegeverfahren an die Lese- und / oder Schreibvorrichtung (11) zu übertragen, wenn die Reinigungs- oder Pflegevorrichtung (1 A, 1 B, 1C) das Reinigungs- oder Pflegeverfahren tatsächlich ausführt.

10. Reinigungs- oder Pflegeeinheit umfassend eine Reinigungs- oder Pflegevorrichtung (1A, 1 B, 1C) nach einem der vorherstehenden Ansprüche und zumindest ein durch die Öffnung (8) der Reinigungs- oder Pflegevorrichtung (1A, 1B, 1C) bewegbares und mit einem durch die Lese- und / oder Schreibvorrichtung (11) auslesbaren und / oder beschreibbaren Datenspeicher (12A, 12B, 12C) versehenes Element, wobei das Element als medizinisches oder dentales Instrument (2, 3), als Träger (15) für ein medizinisches oder dentales Instrument (2, 3) oder als Behältnis (16) für ein medizinisches oder dentales Instrument (2, 3) ausgebildet ist.

11. Verfahren zum Betrieb einer Reinigungs- oder Pflegevorrichtung (1A, 1B, 1C) nach einem der Ansprüche 1 - 9 oder einer Reinigungs- oder Pflegeeinheit nach Anspruch 10, **dadurch gekennzeichnet, dass**
während des Bewegens des Datenspeichers (12A, 12B, 12C) durch die am Gehäuse (4) vorgesehene Öffnung (8) oder in Richtung der Öffnung (8) oder von der Öffnung (8) weg oder während des Vorbeiführens des Datenspeichers (12A, 12B, 12C) am geöffneten Deckel (7) oder während des Vorbeiführens des Datenspeichers (12A, 12B, 12C) an dem die Frontseite (4A) überragenden Vorsprung (13) die Lese- und / oder Schreibvorrichtung (11) den Datenspeicher (12A, 12B, 12C) berührungslos ausliest und / oder beschreibt.

12. Verfahren zum Betrieb einer Reinigungs- oder Pflegevorrichtung (1A, 1B, 1C) nach Anspruch 11, **dadurch gekennzeichnet, dass**
das Bewegen des Datenspeichers (12A, 12B, 12C) relativ zur am Gehäuse (4) vorgesehene Öffnung (8) oder das Vorbeiführen des Datenspeichers (12A, 12B, 12C) am geöffneten Deckel (7) oder das Vorbeiführen des Datenspeichers (12A, 12B, 12C) an dem die Frontseite (4A) überragenden Vorsprung (13) manuell erfolgt.

13. Verfahren zum Betrieb einer Reinigungs- oder Pflegevorrichtung (1 A, 1 B, 1 C) nach Anspruch 11 oder 12, **dadurch gekennzeichnet, dass**
der Datenspeicher (12A, 12B, 12C) beim Einbringen des Datenspeichers (12A, 12B, 12C) in den Reinigungs- oder Pflegeraum (5) ausgelesen wird und dass der Datenspeicher (12A, 12B, 12C) beim Entnehmen des Datenspeichers (12A, 12B, 12C) aus dem Reinigungs- oder Pflegeraum (5) beschrieben wird.

14. Verfahren zum Betrieb einer Reinigungs- oder Pflegevorrichtung (1A, 1B, 1C) nach einem der Ansprüche 11, 12 oder 13, **dadurch gekennzeichnet, dass**
eine mit der Lese- und / oder Schreibvorrichtung (11) verbundene Steuervorrichtung (14) ein von der Reinigungs- oder Pflegevorrichtung (1A, 1 B, 1 C) ausgeführtes Reinigungs- oder Pflegeverfahren überwacht und nur dann Daten über das Reinigungs- oder Pflegeverfahren an die Lese- und / oder Schreibvorrichtung (11) überträgt, wenn die Reinigungs- oder Pflegevorrichtung (1A, 1 B, 1C) das Reinigungs- oder Pflegeverfahren tatsächlich ausführt.

## Claims

1. A cleaning or care device (1A, 1 B, 1 C) for at least one medical or dental instrument (2, 3), comprising a housing (4), a cleaning or care space (5) which is arranged in the interior of the housing (4) to receive the at least one medical or dental instrument (2, 3), a supply device (6) for conveying at least one cleaning or care medium into the cleaning or care space (5) and an opening (8) which is provided on the housing (4), is connected to the cleaning or care space (5), can be sealed by a cover (7) and which is provided for introducing the at least one medical or dental instrument (2, 3) into the cleaning or care space (5) and for removing the at least one medical or dental instrument (2, 3) from the cleaning or care space (5), wherein a coil (9) or an antenna (10) of a reading and/or writing device (11) is provided on the cover (7) or at the opening (8), this coil (9) or antenna (10) being designed to read out from and/or to write to a data memory (12A, 12B, 12C) contactlessly and by means of electromagnetic signals in the radio wavelength range, this data memory (12A, 12B, 12C) being movable through the opening (8), wherein the cover (7) is movable relative to the opening (8), and when the cover (7) closes the opening (8) it has a first side (7A) facing the cleaning or care space (5), wherein the coil (9) or antenna (10) is arranged on said first side (7A), **characterized in that**
the coil (9) or antenna (10) surrounds at least one section of the outer edge (8A) of the opening (8) and wherein the coil (9) or antenna (10) surrounds the opening (8) in a circular or elliptical shape.

2. A cleaning or care device (1A, 1 B, 1 C) for at least one medical or dental instrument (2, 3), comprising a housing (4) with a front side (4A), a cleaning or care space (5) arranged in the interior of the housing (4) for receiving the at least one medical or dental instrument (2, 3), a supply device (6) for conveying at least one cleaning or care medium into the cleaning or care space (5), an opening (8) which is provided on the front side (4A) of the housing (4) and is connected to the cleaning or care space (5) for introducing the at least one medical or dental instrument (2, 3) into the cleaning or care space (5) and for removing the at least one medical or dental instrument (2, 3) from the cleaning or care space (5) and a cover (7) for closing the opening (8) which is movable relative to the walls of the housing (4), **characterized in that**
a coil (9) or an antenna (10) of a reading and/or writing device (11) is provided on a protrusion (13) protruding beyond the front side (4A) and is designed to read out from and/or write to a data memory (12A, 12B, 12C) which is movable through the opening (8) contactlessly and by means of electromagnetic signals in the radio wavelength range.
A

3. The cleaning or care device (1A, 1B, 1C) according to Claim 1 or 2, **characterized in that**
a plurality of coils (9) or a plurality of antenna (10) or at least one coil (9) and one antenna (10) are provided on the opening (8) or on the cover (7) or on the protrusion (13), wherein the coils (9) and/or the antennas (10) have different directivities and/or are designed for transmitting different frequency ranges, or that at least one coil (9) and/or one antenna (10) is/are provided in at least two of the three positions: opening (8), cover (7) or protrusion (13), wherein the coils (9) and/or antennas (10) again have different directivities and/or are designed for transmitting different frequency ranges.

4. The cleaning or care device (1A, 1B, 1C) according to any one of the preceding claims, **characterized in that**
the coil (9) or antenna (10) is provided in or on the housing (4).

5. The cleaning or care device (1A, 1 B, 1C) according to Claim 1, **characterized by** a cover (7) which is movable relative to the opening (8) and, when it closes the opening (8), it has a first side (7A) facing the cleaning or care space (5), wherein the coil (9) or antenna (10) is arranged on this first side (7A).

6. The cleaning or care device (1A, 1B, 1C) according to any one of the preceding claims, **characterized in that**
the reading and/or writing device (11) is designed for inductive energy transfer to the data memory (12A, 12B, 12C).

7. The cleaning or care device (1A, 1B, 1C) according to any one of the preceding claims, **characterized in that**
the reading and/or writing device (11) is connected to a control device (14) which is designed to operate the cleaning or care device (1A, 1 B, 1 C) on the basis of data read out from the data memory (12A, 12B, 12C).

8. The cleaning or care device (1A, 1B, 1C) according to any one of the preceding claims, **characterized in that**
the reading and/or writing device (11) is connected to a control device (14) which is designed to transmit data about a cleaning or care method performed by the cleaning or care device (1A, 1B, 1C) to the reading and/or writing device (11), so that the reading and/or writing device (11) transmits these data to the data memory (12A, 12B, 12C).

9. The cleaning or care device (1A, 1 B, 1 C) according to Claim 8, **characterized in that**
the control device (14) is designed to monitor the cleaning or care method and to transmit data about the cleaning or care method to the reading and/or writing device (11) only when the cleaning or care device (1A, 1B, 1C) is actually carrying out the cleaning or care method.

10. A cleaning or care unit, comprising a cleaning or care device (1A, 1B, 1C) according to any one of the preceding claims and at least one element which is movable through the opening (8) of the cleaning or care device (1A, 1 B, 1C) and is provided with a data memory (12A, 12B, 12C) that can be read out and/or written to by the reading and/or writing device (11), wherein the element is designed as a medical or dental instrument (2, 3), as a carrier (15) for a medical or dental instrument (2, 3) or as a container (16) for a medical or dental instrument (2, 3).

11. A method for operating a cleaning or care device (1A, 1 B, 1 C) according to any one of Claims 1 to 9 or a cleaning or care unit according to Claim 10, **characterized in that** during the movement of the data memory (12A, 12B, 12C) through the opening (8) provided on the housing (4) or in the direction of the opening (8) or away from the opening (8) or during the passage of the data memory (12A, 12B, 12C) past the opened cover (7) or during the passage of the data memory (12A, 12B, 12C) past the protrusion (13) that protrudes beyond the front side (4A) the reading and/or writing device (11) reads out from and/or writes to the data memory (12A, 12B, 12C) contactlessly.

12. The method for operating a cleaning or care device (1A, 1 B, 1 C) according to Claim 11, **characterized in that**
the movement of the data memory (12A, 12B, 12C) relative to the opening (8) which is provided on the housing (4) or the passage of the data memory (12A, 12B, 12C) past the opened cover (7) or the passage of the data memory (12A, 12B, 12C) past the protrusion (13) that protrudes beyond the front side (4A) is done manually.

13. The method for operating a cleaning or care device (1A, 1 B, 1 C) according to Claim 11 or 12, **characterized in that**
the data memory (12A, 12B, 12C) is read out when the data memory (12A, 12B, 12C) is introduced into the cleaning or care space (5), and the data memory (12A, 12B, 12C) is written when the data memory (12A, 12B, 12C) is removed from the cleaning or care space (5).

14. The method for operating a cleaning or care device (1A, 1 B, 1 C) according to any one of Claims 11, 12 or 13, **characterized in that**
a control device (14) which is connected to the reading and/or writing device (11) monitors a cleaning or care method which is carried out by the cleaning or care device (1A, 1B, 1C) and transmits data about the cleaning or care method to the reading and/or writing device (11) when the cleaning or care device (1A, 1B, 1C) actually carries out the cleaning or care method.

## Revendications

1. Dispositif de nettoyage ou d'entretien (1A, 1B, 1C) pour au moins un instrument médical ou dentaire (2, 3), comprenant un boîtier (4), un espace de nettoyage ou d'entretien (5) disposé à l'intérieur du boîtier (4) pour réceptionner cet au moins un instrument médical ou dentaire (2, 3), un dispositif d'alimentation (6) pour l'introduction d'au moins un milieu de nettoyage ou d'entretien dans l'espace de nettoyage ou d'entretien (5) et une ouverture (8) prévue sur le boîtier (4), reliée à l'espace de nettoyage ou d'entretien (5) et pouvant être fermée par un couvercle (7) pour l'introduction de cet au moins un instrument médical ou dentaire (2, 3) dans l'espace de nettoyage ou d'entretien (5) et pour le prélèvement de cet au moins un instrument médical ou dentaire (2, 3) de l'espace de nettoyage ou d'entretien (5), dans lequel on prévoit, sur le couvercle (7) ou sur l'ouverture (8), une bobine (9) ou une antenne (10) d'un dispositif de lecture et/ou d'écriture (11) qui est réalisée pour la lecture sans contact d'une mémoire de données (12A, 12B, 12C) et/ou l'écriture sans contact dans cette mémoire de données (12A, 12B, 12C) déplaçable à travers l'ouverture (8) au moyen de signaux électromagnétiques dans la plage des ondes radio, dans lequel le couvercle (7) peut être déplacé par rapport à l'ouverture (8) et, lorsqu'il ferme l'ouverture (8), présente une première face (7A) tournée vers l'espace de nettoyage ou d'entretien (5) sur laquelle est disposée la bobine (9) ou l'antenne (10), **caractérisé en ce que**
la bobine (9) ou l'antenne (10) entoure au moins une partie du bord extérieur (8A) de l'ouverture (8), dans lequel la bobine (9) ou l'antenne (10) entoure l'ouverture (8) de manière circulaire ou elliptique.

2. Dispositif de nettoyage ou d'entretien (1A, 1B, 1C) pour au moins un instrument médical ou dentaire (2, 3), comprenant un boîtier (4) avec une face avant (4A), un espace de nettoyage ou d'entretien (5) disposé à l'intérieur du boîtier (4) pour réceptionner cet au moins un instrument médical ou dentaire (2, 3), un dispositif d'alimentation (6) pour l'introduction d'au moins un milieu de nettoyage ou d'entretien dans l'espace de nettoyage ou d'entretien (5), une ouverture (8) prévue sur la face avant (4A) du boîtier (4) et reliée à l'espace de nettoyage ou d'entretien (5) pour l'introduction de cet au moins un instrument médical ou dentaire (2, 3) dans l'espace de nettoyage ou d'entretien (5) et pour le prélèvement de cet au moins un instrument médical ou dentaire (2, 3) de l'espace de nettoyage ou d'entretien (5) et un couvercle (7) pour fermer l'ouverture (8), lequel est déplaçable par rapport aux parois du boîtier (4), **caractérisé en ce que**
l'on prévoit, sur une saillie (13) surplombant la face avant (4A), une bobine (9) ou une antenne (10) d'un dispositif de lecture et/ou d'écriture (11) qui est réalisée pour la lecture sans contact d'une mémoire de données (12A, 12B, 12C) et/ou l'écriture sans contact dans cette mémoire de données (12A, 12B, 12C) déplaçable à travers l'ouverture (8) au moyen de signaux électromagnétiques dans la plage des ondes radio.

3. Dispositif de nettoyage ou d'entretien (1A, 1B, 1C) selon la revendication 1 ou 2, **caractérisé en ce que**
l'on prévoit plusieurs bobines (9) ou plusieurs antennes (10) ou au moins une bobine (9) et une antenne (10) sur l'ouverture (8) ou sur le couvercle (7) ou sur la saillie (13), dans lequel les bobines (9) et/ou antennes (10) présentent des directivités différentes et/ou sont réalisées pour la transmission de plages de fréquences différentes ou bien en ce que l'on prévoit au niveau d'au moins deux des trois positions: ouverture (8), couvercle (7) ou saillie (13), respectivement au moins une bobine (9) et/ou une antenne (10), dans lequel les bobines (9) et/ou antennes (10) présentent à leur tour des directivités différentes et/ou sont réalisées pour la transmission de plages de fréquences différentes.

4. Dispositif de nettoyage ou d'entretien (1A, 1B, 1C) selon l'une des revendications précédentes, **caractérisé en ce que**
la bobine (9) ou l'antenne (10) est prévue dans ou sur le boîtier (4).

5. Dispositif de nettoyage ou d'entretien (1A, 1B, 1C) selon la revendication 1, **caractérisé par**
un couvercle (7) déplaçable par rapport à l'ouverture (8) lequel, lorsqu'il ferme l'ouverture (8), présente une première face (7A) tournée vers le dispositif de nettoyage ou d'entretien (5), dans lequel la bobine (9) ou l'antenne (10) est disposée sur cette première face (7A).

6. Dispositif de nettoyage ou d'entretien (1A, 1B, 1C) selon l'une des revendications précédentes, **caractérisé en ce que**
le dispositif de lecture et/ou écriture (11) est réalisé pour la transmission inductive d'énergie à la mémoire de données (12A, 12B, 12C).

7. Dispositif de nettoyage ou d'entretien (1A, 1B, 1C) selon l'une des revendications précédentes, **caractérisé en ce que**
le dispositif de lecture et/ou écriture (11) est relié à un dispositif de commande (14), lequel est réalisé pour exploiter le dispositif de nettoyage ou d'entretien (1A, 1 B, 1 C) sur la base des données lues dans la mémoire de données (12A, 12B, 12C).

8. Dispositif de nettoyage ou d'entretien (1A, 1B, 1C) selon l'une des revendications précédentes, **caractérisé en ce que**
le dispositif de lecture et/ou écriture (11) est relié à un dispositif de commande (14) qui est réalisé pour transmettre des données sur un processus de nettoyage ou d'entretien exécuté par le dispositif de nettoyage ou d'entretien (1A, 1B, 1C) à l'attention du dispositif de lecture et/ou écriture (11) de manière à ce que le dispositif de lecture et/ou écriture (11) transmette ces données à la mémoire de données (12A, 12B, 12C).

9. Dispositif de nettoyage ou d'entretien (1A, 1B, 1C) selon la revendication 8, **caractérisé en ce que**
le dispositif de commande (14) est réalisé pour surveiller le processus de nettoyage ou d'entretien et pour seulement transmettre des données sur le processus de nettoyage ou d'entretien à l'attention du dispositif de lecture et/ou écriture (11) si le dispositif de nettoyage ou d'entretien (1A, 1B, 1C) exécute effectivement le processus de nettoyage ou d'entretien.

10. Unité de nettoyage ou d'entretien comprenant un dispositif de nettoyage ou d'entretien (1A, 1B, 1C) selon l'une des revendications précédentes et au moins un élément déplaçable à travers l'ouverture (8) du dispositif de nettoyage ou d'entretien (1A, 1B, 1C) et comprenant une mémoire de données (12A, 12B, 12C) pouvant être lue avec le dispositif de lecture et/ou écriture (11) et/ou dans laquelle mémoire on peut écrire grâce à celui-ci, dans lequel l'élément est réalisé en tant qu'instrument médical ou dentaire (2, 3), en tant que support (15) pour un instrument médical ou dentaire (2, 3) ou en tant que récipient (16) pour un instrument médical ou dentaire (2, 3).

11. Procédé pour l'exploitation d'un dispositif de nettoyage ou d'entretien (1A, 1B, 1C) selon l'une des revendications 1-9 ou d'une unité de nettoyage ou d'entretien selon la revendication 10, **caractérisé en ce que**
pendant le déplacement de la mémoire de données (12A, 12B, 12C) à travers l'ouverture (8) prévue sur le boîtier (4) ou en direction de l'ouverture (8) ou en éloignement de l'ouverture (8) ou pendant que l'on fait passer la mémoire de données (12A, 12B, 12C) à côté du couvercle (7) ouvert ou pendant que l'on fait passer la mémoire de données (12A, 12B, 12C) à côté de la saillie (13) surplombant la face frontale (4A), le dispositif de lecture et/ou écriture (11) lit la mémoire de données (12A, 12B, 12C) de manière sans contact et/ou écrit dans celle-ci.

12. Procédé pour l'exploitation d'un dispositif de nettoyage ou d'entretien (1A, 1 B, 1 C) selon la revendication 11, **caractérisé en ce que**
le déplacement de la mémoire de données (12A, 12B, 12C) par rapport à l'ouverture (8) prévue sur le boîtier (4) ou le fait de faire passer la mémoire de données (12A, 12B, 12C) à côté du couvercle (7) ouvert ou le fait de faire passer la mémoire de données (12A, 12B, 12C) à côté de la saillie (13) surplombant la face frontale (4A) s'effectue manuellement.

13. Procédé pour l'exploitation d'un dispositif de nettoyage ou d'entretien (1A, 1 B, 1 C) selon la revendication 11 ou 12, **caractérisé en ce que**
la mémoire de données (12A, 12B, 12C) est lue lors de l'introduction de la mémoire de données (12A, 12B, 12C) dans l'espace de nettoyage ou d'entretien (5) et **en ce que** l'on écrit dans la mémoire de données (12A, 12B, 12C) lors du prélèvement de la mémoire de données (12A, 12B, 12C) de l'espace de nettoyage ou d'entretien (5).

14. Procédé pour l'exploitation d'un dispositif de nettoyage ou d'entretien (1A, 1 B, 1 C) selon l'une des revendications 11, 12 ou 13, **caractérisé en ce qu'**un dispositif de commande (14) relié au dispositif de lecture et/ou écriture (11) surveille un processus de nettoyage ou d'entretien exécuté par le dispositif de nettoyage ou d'entretien (1A, 1B, 1C) et transmet seulement des données sur le processus de nettoyage ou d'entretien à l'attention du dispositif de lecture et/ou écriture (11) si le dispositif de nettoyage ou d'entretien (1A, 1B, 1C) exécute effectivement le processus de nettoyage ou d'entretien.
